# EUROPEAN PATENT APPLICATION

(11) **EP 1 527 741 A1**
(43) Date of publication of application: **04.05.2005**
(21) Application number: 03425709.7
(22) Date of filing: 31.10.2003
(51) Int. Cl.: A61B 17/58, A61B 17/56, A61B 17/68, A61B 17/86, A61B 17/04

(54) **Insert for orthopaedic use**

(71) Applicant: ORTHOFIX INTERNATIONAL B.V., 1071 Amsterdam (NL)
(72) Inventor: Venturini, Daniele, 37064 Povegliano Veronese (Verona) (IT); Corradi, Maurizio, 43100 Parma (IT); Rossi, Luigi, 37019 Peschiera del Garda (IT)
(74) Representative: Botti, Mario

(57) **Abstract**

The invention relates to an insert (10) for orthopaedic use that has advantageously uncommon expansion properties; the insert (10) comprises a jarlike body including a substantially cylindrical skirt (12) with a longitudinal axis (X-X), wherein said jarlike body is closed at one end (14) by an end wall (15) and open at an opposite end (16), where an opening (17) gives access to a substantially cylindrical cavity (18) defined in said skirt (12), in said cavity (18) a shank can be inserted producing a deformation of at least a region (22) of said skirt (12) in a radial outward direction with respect to the axis (X-X) of said insert (10), at least one slit (36) being formed through at least one portion of the skirt (12), said at least one slit (36) communicating said cavity (18) to the skirt (12) exterior and extending substantially lengthwise of the skirt (12).

## Description

### Field of Application

The present invention relates, in its more general aspect, to an insert for orthopaedic use.

In particular, this invention relates to an insert comprising a jarlike body having a skirt and being closed at one end by an end wall and open at an opposite end, where an opening gives access to a cavity defined internally of said skirt.

A typical exemplary application of such inserts concerns articular prostheses, i.e. mechanical devices used for restoring the function of a damaged articulation. In this case, the insert would be secured in the medullary canal of a bone and connected to an articulated joint half part of the articular prosthesis, the articulated joint being adapted to restore the function of the damaged articulation.

### Prior Art

Inserts have been known which are intended for installation inside the medullary canal of a bone, following careful shaping of same, and are secured there by expansion of at least a region of the insert, so that the insert can fit snugly inside the canal in direct contact with the bone. Later on, a porous coat provided on the insert surface is osteointegrated with the bone as bone tissue is allowed to grow over the installed insert and hold the insert securely in place.

As a rule, such inserts come in the form of a jarlike body comprising a substantially cylindrical skirt with a preferably frusto-conical outline of small flare angle. The smaller base of this frustum of cone is closed by a circular end wall, at the opposite base of this frustum of cone the body being open by way of an opening that gives access to a substantially cylindrical cavity defined by the skirt. A shank is installed into said cavity. The installed shank has a deforming effect causing at least a region of the insert skirt to expand in radial directions with respect to the insert axis.

Inserts are required to have excellent mechanical properties, and must be bio-compatible: in orthopaedics, titanium or chromium-cobalt alloys are generally used.

For the insert to be expanded as required, a first prior proposal teaches the use of small thicknesses: however, in this case, cracks are likely to develop through thin insert walls and allow bone tissue to grow undesirably into the insert, which may result in the insert taking an offset from the desired axis, that is used during the preparatory stage of thebone medullary canal. Another drawback brought about by bone growth into the insert is that a shank which has been installed cannot be recovered, for example in a possible operation after the implantation of the prosthesis.

A second prior art proposal teaches lightening the material inareas of the skirt, as by a series of longitudinal grooves in the walls of the frusto-conical outline. Again, cracks may develop along such grooves and allow bone tissue to grow into the insert.

### Summary of the Invention

The underlying problem of this invention is to provide an insert for orthopaedic use, which can overcome the aforementioned drawbacks of the prior art.

This problem is solved by the present invention providing an insert for orthopaedic use, as previously indicated, which is characterized in that at least one slit is formed through at least one portion of the skirt, said at least one slit communicating said cavity to the skirt exterior and extending substantially lengthwise of the skirt.

Further features and the advantages of an insert for orthopaedic use, according to this invention, will be apparent from the following description of an embodiment thereof, given by way of non-limitative example with reference to the attached drawings.

### Brief Description of the Drawings

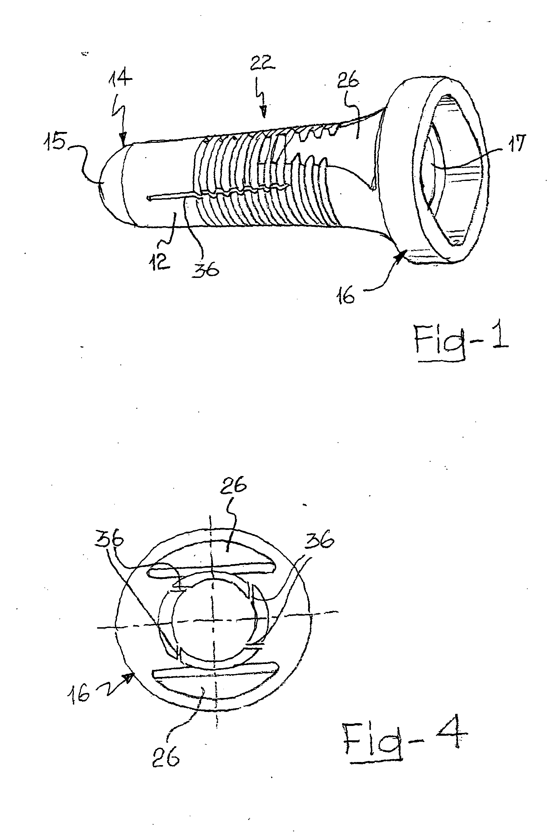

Figure 1 is a schematic perspective view of an insert for orthopaedic use according to the invention.

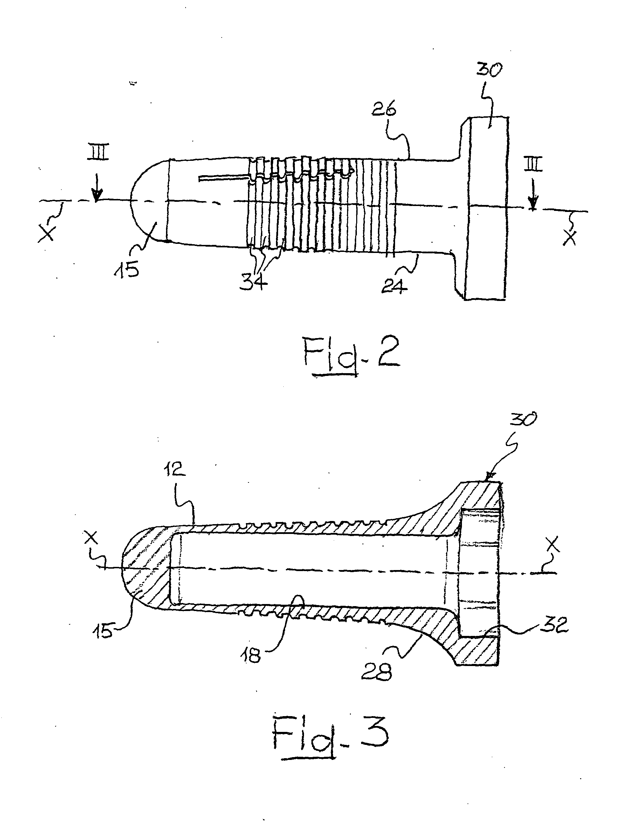

Figure 2 is a schematic plan view of the insert of Figure 1.

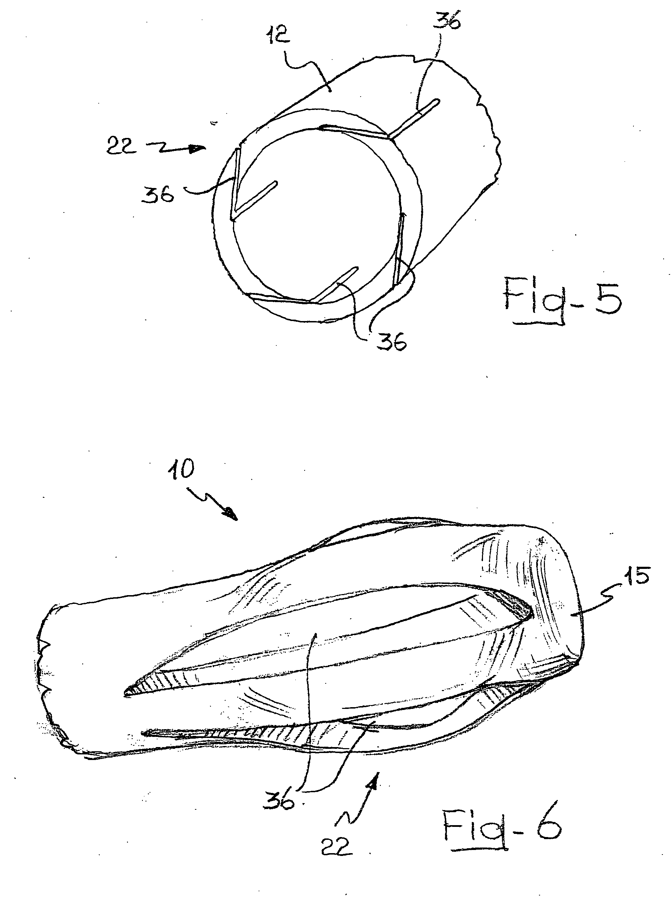

Figure 3 is a sectional view of the insert of Figure 1 taken according to the traced III-III plane of Figure 2.
Figure 4 is a front elevation view of the insert of Figure 1.
Figure 5 is a schematic perspective view of a portion of the insert shown in Figure 1.
Figure 6 is a perspective view of the insert of Figure 1 in an expanded condition.

### Detailed Description of a Preferred Embodiment

With reference to the drawings, an insert for orthopaedic use according to this invention is shown and is globally indicated with 10.

The insert 10 is usually intended for installation in the medullary canal of a bone, following careful shaping of same, the insert 10 having a longitudinal axis X-X and a jarlike body comprising a substantially cylindrical skirt 12.

The skirt 12 is closed at one end 14 by an end wall 15, and is open at the opposite end 16 through an opening 17 giving access to a cavity 18 defined in the skirt 12. This cavity 18 is commonly of substantially cylindrical shape. A shank (not shown in the drawings) may be installed in the cavity 18, the introduction of the shank causing at least a region 22 of the skirt 10 to expand in a radial direction with respect to the insert axis X-X. In practice, the region 22 forms a weakened region of the skirt 12. The insert 10 is made of a bio-compatible material, such as a titanium or chromium-cobalt alloy. The bone-contacting areas of the insert are coated with hydroxyapatite for its osteointegration with the bone.

The outline of the skirt 12 is flattened at two axially opposed regions 24 and 26. The opening 17 of the insert 10, at an opposite side with respect to the cavity 18, extends, with a conical divarication 28, into a terminating collar 30 of substantially cylindrical outside shape. The collar 30 defines a through opening 32, for example of quadrilateral shape, preferably square, the through opening 32 being larger than the cavity 18 of the insert 10 and being in communication with said cavity 18.

The outer outline of the skirt 12 has circumferential grooves 34. In addition, at least one slit 36 is formed through at least one portion of the skirt 12, and in particular, on the side towards the end 14 closed by the end wall 15, at least one slit 36 is formed, said slit 36communicating the cavity 18 to the exterior of the skirt 12 and extending substantially lengthwise of the skirt 12.

According to one aspect of this invention, the slits 36 lie in slitting planes substantially parallel to the axis X-X of the insert 10 and are substantially tangential of the cavity 18, i.e., in the example shown, the slits extend tangentially of the generatrix circumference of cylinder of the substantially cylindrical cavity 18. Preferably, the slits 36 are equidistant around the circumference, and there may be four such slits provided, for example.

In general, the shank is substantially frusto-conical and flares out toward one end, where it is formed with a substantially cylindrical beat collar. The base end of the collar next to the frusto-conical portion of the shank, is provided with a parallelepipedal body having a suitable cross-sectional shape to be inserted in the through opening 32 of the collar 30 of the insert 10.

The operation of the insert for orthopaedic use according to this invention is now explained.

The insert 10 is first introduced into the medullary canal of the bone.

The shank is then introduced through the opening 17 into the cavity 18 of the insert 10. The frusto-conical portion of the shank conventionally compresses the sidewall 22 of the cavity 18, for example it is provided an interference relationship, and begins to deform a region 22 of the skirt 12 in radial directions. The pressure of the shank against the inner wall of the cavity 18, that is increased until the beat collar of the shank abuts with the terminating collar 30 of the insert 10, causes an expansion of the region 22 of the skirt 12.

This expansion is increased by the slits 36 provided. However, the special layout chosen for the slits 36 prevents large through ports from forming between the insert 10 outside and the insert 10 inside, thus preventing bone tissue from growing into the insert 10. In practice, as an outer portion of the slit 36 opens up, an inner portion of the slit 36 tends to shut itself, thereby occluding the through port of the undeformed slit 36.

It should be noted that, by virtue of a good expansion of the insert, good stability of the inserts 10 employed is achieved immediately after the prosthesis is implanted: a fast rehabilitation is thus allowed. In time, the final osteointegration between the insert 10 and the bone occurs:. this stable anchoring establishes itself at the circumferential grooves 34, the latter being deformed by the expansion undergone by the skirt 12. Accordingly, the growth of bone tissue into the grooves 34 secures the insert 10 firmly in place, especially in the axial direction.

Not to be overlooked, moreover, is that the insert 10 is closed at the front by the end wall 15 to prevent the bone from growing inside. Moreover, the conical divarication 28 of the insert 10, located before the collar 30, prevents undesirable stepwise bone resorptions from forming.

The main advantage of the insert for orthopaedic use according to this invention is that it allows for an uncommon amount of expansion at no risk of the insert sidewall breaking anywhere.

The insert for orthopaedic use just described can undergo some changes, all within the reach of the skilled in the art and falling within the scope of protection of the present invention, as defined by the following claims.

## Claims

1. Insert (10) for orthopaedic use, comprising a jarlike body including a substantially cylindrical skirt (12) with a longitudinal axis (X-X), wherein said jarlike body is closed at one end (14) by an end wall (15) and open at an opposite end (16), where an opening (17) gives access to a substantially cylindrical cavity (18) defined in said skirt (12), in said cavity (18) a shank can be inserted producing a deformation of at least a region (22) of said skirt (12) in a radial outward direction with respect to the axis (X-X) of said insert (10), **characterized in that** at least one slit (36) is formed through at least one portion of the skirt (12), said at least one slit (36) communicating said cavity (18) to the skirt (12) exterior and extending substantially lengthwise of the skirt (12).

2. Insert (10) according to Claim 1, **characterized in that** said slit (36) is formed in a slitting plane lying substantially parallel to the axis (X-X) of said insert (10) and substantially tangential to the cylinder of said substantially cylindrical cavity (18).

3. Insert (10) according to either Claim 1 or 2, **characterized in that** a sequence of said slits (36) is provided, equidistant around the circumference.

4. Insert (10) according to Claim 1, **characterized in that** it is installed in a medullary canal of a bone following careful shaping of same.

5. Insert (10) according to Claim 1, **characterized in that** it is made of a bio-compatible material and coated with hydroxyapatite at its contact areas with the bone.

6. Insert (10) according to Claim 1, **characterized in that** the skirt (12) exterior is flattened at two axially opposed regions (24,26).

7. Insert (10) according to Claim 1, **characterized in that** said opening (17) on an opposite side with respect to the cavity (18), extends, with a conical divarication (28), into a terminating collar (30) of substantially cylindrical shape.

8. Insert (10) according to Claim 7, **characterized in that** said collar (30) is formed with a through opening (32) that is larger than the cavity (18) of the insert (10), said through opening (32) being in communication with said cavity (18).

9. Insert (10) according to Claim 8, **characterized in that** said through opening (32) has a quadrilateral shape.

10. Insert (10) according to Claim 1, **characterized in that** the said skirt (12) externally has circumferential grooves (34).

11. Insert (10) according to Claim 1, **characterized in that** said at least one portion of the skirt (12) locates on a side towards the end (14) closed by the end wall (15).

12. Insert (10) according to Claim 1, **characterized in that** said shank has a substantially frusto-conical shape flaring out toward an end formed with a substantially cylindrical beat collar.

13. Insert (10) according to Claims 9 and 12, **characterized in that** the base end of said collar, next to the frusto-conical portion of the shank, is provided with a parallelepipedal body having a suitable cross-sectional shape to be inserted in the through opening (32) of the collar (30) of the insert (10).
